# EUROPÄISCHE PATENTANMELDUNG

(11) **EP 1 132 108 A1**
(43) Veröffentlichungstag der Anmeldung: **12.09.2001**
(21) Anmeldenummer: 01810154.3
(22) Anmeldetag: 16.02.2001
(51) Int. Cl.: A61M 39/28, F16K 7/04, F16K 31/08

(54) **Klemmvorrichtung**

(30) Priorität: 06.03.2000 EP 00810184
(71) Anmelder: Levitronix LLC, Waltham, MA 02451 (US)
(72) Erfinder: Schöb, Reto, Dr., 8604 Volketswil (CH)
(74) Vertreter: Sulzer Management AG

(57) **Zusammenfassung**

Es wird eine Klemmvorrichtung zum Abklemmen eines Schlauchs in einem Fluidsystem für biologische Fluide, insbesondere Blut, vorgeschlagen, mit einem bewegbar angeordneten Schliessorgan (3), welches ein Schliessstück (31,31') zum Abklemmen des Schlauches (10) umfasst, mit einer permanentmagnetischen Halteeinrichtung (2), welche derart angeordnet und ausgestaltet ist, dass sie das Schliessorgan (3) gegen eine Kraft in zwei verschiedenen stabilen Gleichgewichtslagen, nämlich einer Offenstellung und einer Schliessstellung, halten kann, ohne dass der permanentmagnetischen Halteeinrichtung (2) für das Halten in der jeweiligen Gleichgewichtslage Energie zugeführt werden muss, sowie mit Betätigungsmitteln (4;4a,4b;33;33'), um das Schliessorgan (3) aus der Offenstellung in die Schliessstellung zu bewegen.

## Beschreibung

Die Erfindung betrifft eine Klemmvorrichtung zum Abklemmen eines Schlauchs in einem Fluidsystem für biologische Flüssigkeiten, insbesondere Blut, gemäss dem Oberbegriff des unabhängigen Anspruchs 1.

Fluidsysteme für biologische Flüssigkeiten umfassen typischerweise eine Pumpvorrichtung für das zu fördernde Fluid, welche über Schläuche mit einem Kreislauf bzw. mit anderen Elementen des Fluidsystems verbunden sind. Als Beispiel für eine solches Fluidsystem seien hier Herz-Lungen-Maschinen genannt, welche z. B. während einer Herzoperation mit dem Blutkreislauf des Patienten verbunden werden, um die Funktion des Herzens zu übernehmen und den Blutkreislauf aufrechtzuerhalten. Dabei ist es sehr wichtig, dass in dem Blut, welches in den Kreislauf des Patienten gefördert wird, möglichst keine Luftblasen vorhanden sind, weil diese ernsthafte Gefährdungen des Patienten darstellen. Daher ist in Herz-Lungen-Maschinen üblicherweise stromabwärts der Pumpe ein Blasendetektor und eine Klemmvorrichtung vorgesehen. Sobald der Blasendetektor eine Luftblase detektiert, muss die Klemmvorrichtung möglichst schnell den Schlauch, durch welchen das geförderte Blut in den Körper des Patienten strömt, abklemmen und damit die Blutversorgung zum Patienten unterbrechen, damit die Luftblase nicht in den Körperkreislauf vordringen kann. Die Klemmvorrichtung dient somit als Ein/Aus-Schalter für die Strömungsverbindung zwischen der Blutpumpe und dem Körperkreislauf des Patienten. Damit die Klemmvorrichtung die Strömungsverbindung genügend schnell durch Abklemmen des Schlauchs unterbrechen kann, ist es wünschenswert, dass ihre Schaltzeit weniger als 100 Millisekunden, vorzugsweise höchstens 80 Millisekunden beträgt.

Es sind Klemmvorrichtungen bekannt, bei denen das Abklemmen mittels einer Spindel und einem die Spindel antreibenden Schrittmotor erfolgt. Diese Vorrichtungen haben jedoch den Nachteil, dass sie konstruktiv aufwendig sind und dass für eine ausreichende Schnelligkeit relativ grosse und leistungsstarke Schrittmotoren notwendig sind. Zudem sind solche Klemmvorrichtungen durch den Spindelantrieb bedingt selbsthemmend. Im Falle eines Ausfalls der Elektrik ist es daher - wenn überhaupt - nur sehr schwer möglich, die Klemmvorrichtung manuell zu öffnen oder zu schliessen.

Ferner sind Klemmvorrichtungen bekannt, bei welchen das Schliessorgan, welches den blutführenden Schlauch abklemmt, elektromagnetisch betätigt wird. Das Schliessorgan wird mittels eines Elektromagneten, meist gegen die Kraft einer Feder, in die Schliessstellung bewegt und dort gehalten, oder umgekehrt, durch einen Elektromagneten gegen die Kraft einer Feder in der Offenstellung gehalten. Durch Deaktivieren des Elektromagneten bewegt sich dann das Schliessorgan aufgrund der Federkraft je nach konktreter Ausgestaltung in die Offenstellung oder in die Schliessstellung. Nachteilig an solchen Klemmvorichtungen ist ihr hoher Energieverbrauch, denn zumindest für einen Haltezustand, nämlich das Halten des Schliessorgans in der Offenstellung oder das Halten in der Schliessstellung wird ständig Energie benötigt, um den Elektromagneten mit Strom zu versorgen. Der ständige Stromfluss führt ferner zu einer nachteiligen Wärmeentwicklung. Der hohe Energiebedarf ist insbesondere bei tragbaren bzw. mobilen Systemen ein erheblicher Nachteil, weil solche Systeme üblicherweise mit Batterien gespeist werden. Auch im Hinblick auf Situationen, in denen die Herz-Lungen-Maschine nur mit Notstromversorgung betrieben werden kann, ist es wünschenswert, den Strombedaf möglichst gering zu halten. Zudem haben solche elektromagnetisch betriebenen Klemmvorrichtungen auch den Nachteil, dass sie beim Ausfall der Elektrik nicht manuell geöffnet oder geschlossen werden können.

Ausgehend von diesem Stand der Technik ist es daher eine Aufgabe der Erfindung, eine Klemmvorrichtung zum Abklemmen eines Schlauches in einem Fluidsystem für biologische Flüssigkeiten vorzuschlagen, welche diese Nachteile nicht aufweist. Insbesondere soll die Klemmvorrichtung während des Betriebs möglichst wenig Energie (Strom) benötigen, konstruktiv einfach und möglichst kompakt sein. Sie soll ausreichend schnelle Schaltzeiten von weniger als etwa 100 Millisekunden ermöglichen, und zudem in einfacher Weise manuell betätigbar sein.

Die diese Aufgaben lösende Klemmvorrichtung ist durch die Merkmale des unabhängigen Anspruchs 1 gekennzeichnet.

Erfindungsgemäss wird also eine Klemmvorrichtung zum Abklemmen eines Schlauchs in einem Fluidsystem für biologische Fluide, insbesondere Blut, vorgeschlagen, mit einem bewegbar angeordneten Schliessorgan, welches ein Schliessstück zum Abklemmen des Schlauches umfasst, mit einer permanentmagnetischen Halteeinrichtung, welche derart angeordnet und ausgestaltet ist, dass sie das Schliessorgan gegen eine Kraft in zwei verschiedenen stabilen Gleichgewichtslagen, nämlich einer Offenstellung und einer Schliessstellung, halten kann, ohne dass der permanentmagnetischen Halteeinrichtung für das Halten in der jeweiligen Gleichgewichtslage Energie zugeführt werden muss, sowie mit Betätigungsmitteln, um das Schliessorgan aus der Offenstellung in die Schliessstellung zu bewegen.

Die permanentmagnetische Halteeinrichtung ist also derart ausgestaltet, dass für das Schliessorgan zwei stabile Gleichgewichtslagen existieren, nämlich zum einen die Offenstellung, in welcher ein in die Klemmvorrichtung eingelegter Schlauch nicht oder nur wenig geklemmt wird, sodass die Flüssigkeit durch den Schlauch hindurchströmen kann, und zum anderen eine Schliessstellung, in welcher der Schlauch durch das Schliessstück des Schliessorgans abgeklemmt wird, sodass keine Flüssigkeit mehr durch den Schlauch strömen kann. Zum Halten des Schliessorgans in den beiden Gleichgewichtslagen bedarf es keiner Energie in Form von Strom. Das Schliessorgan wird in den beiden Gleichgewichtslagen rein passiv, nämlich permanentmagnetisch, gehalten, was im Hinblick auf den Energieverbrauch ein ganz erheblicher Vorteil ist.

Zudem sind keine Spindelantriebe oder sonstige selbsthemmenden Antriebe zur Betätigung der Klemmvorrichtung notwendig, weshalb die Klemmvorrichtung konstruktiv einfach, sehr kompakt und insbesondere auch manuell betätigbar ist, das heisst manuell in die Offenstellung und in die Schliessstellung gebracht werden kann.

Vorzugsweise umfassen die Betätigungsmittel mindestens eine Spule, welche so angeordnet ist, dass sie auf das Schliessorgan eine in Richtung auf die Schliessstellung oder in Richtung auf die Offenstellung wirkende elektomagnetische Kraft ausüben kann. Durch Aktivieren der Spule wird zusätzlich zur permanentmagnetischen Haltekraft eine elektromagnetische Kraft erzeugt, welche das Schliessorgan so weit aus seiner einen stabilen Gleichgewichtslage auslenkt, dass es seine andere stabile Gleichgewichtslage einnimmt. Die Spule braucht also nur aktiviert zu werden, falls das Schliessorgan aus der Offen- in die Schliessstellung gebracht werden soll oder umgekehrt aus der Schliess- in die Offenstellung.

Bei einem bevorzugten Ausführungsbeispiel umfasst das Schliessorgan zwei Schenkel sowie eine Querstange, wobei die Schenkel beabstandet zueinander mit der Querstange verbunden sind. Die permanentmagnetische Halteeinrichtung umfasst dann zwei permanentmagnetische Halter, von denen jeder jeweils einen Schenkel umgibt, und bei welcher das Schliessstück an der Querstange zwischen den beiden Schenkeln angeordnet ist. Vorzugsweise ist ferner für jeden Schenkel des Schliessorgans mindestens eine den Schenkel umgebende Spule zum Ausüben einer elektromagnetischen Kraft auf den Schenkel vorgesehen. Dieses Ausführungsbeispiel zeichnet sich durch sein besonders sicheres Betriebsverhalten aus.

Vorzugsweise sind für das Schliessorgan oder für jeden Schenkel des Schliessorgans zwei separate Spulen als Betätigungsmittel vorgesehen, wobei für jede der beiden separaten Spulen jeweils eine getrennte Ansteuerund Versorgungsvorrichtung vorgesehen ist. Durch diese Massnahme lässt sich eine vorteilhafte Fehlertoleranz realisieren, welche die Betriebssicherheit erhöht. Falls nämlich eine der beiden separaten Spulen oder eine der beiden Ansteuer- und Versorgungsvorrichtungen aufgrund eines Fehlers ausfällt, beispielsweise durch den Bruch einer Leitung bzw. eines Kabels oder einen sonstigen Schaden, kann das Schliessorgan mit der noch fehlerfreien Spule weiterhin elektromagnetisch betätigt werden, sodass die Klemmvorrichtung vollständig funktionstüchtig bleibt.

Eine weitere vorteilhafte Massnahme besteht darin, mindestens ein auf das Schliessorgan einwirkendes Federelement vorzusehen, welches so angeordnet ist, dass die Federkraft der magnetischen Kraft entgegenwirkt, welche die permanentmagnetische Haltevorrichtung auf das Schliessorgan ausübt. Durch diese Massnahme lässt sich die Klemmvorrichtung in einfacher Weise an die Eigenschaften des jeweils verwendeten Schlauchs anpassen.

Die erfindungsgemässe Klemmvorrichtung eignet sich insbesondere zur Kombination mit einer Pumpvorrichtung zum Fördern eines biologischen Fluids, speziell zur Kombination mit einer Blutpumpe, beispielsweise in einer Herz-Lungen-Maschine.

Weitere vorteilhafte Massnahmen und bevorzugte Ausgestaltungen der Erfindung ergeben sich aus den abhängigen Ansprüchen.

Im Folgenden wird die Erfindung anhand von Ausführungsbeispielen und anhand der Zeichnung näher erläutert. In der teilweise schematischen Zeichnung zeigen:
- Fig. 1:: Einen Längsschnitt durch ein erstes Ausführungbeispiel der erfindungsgemässen Klemmvorrichtung,
- Fig.2:: die permanentmagnetische Halteeinrichtung aus Fig. 1 (ohne Schliessorgan)
- Fig. 3:: ein Diagramm mit Kraft-Weg-Kennlinien,
- Fig. 4:: wie Fig. 3, jedoch inklusive Kennlinien für das Schalten in die Schliessstellung bzw. in die Offenstellung,
- Fig. 5:: eine Ausführungsform von Sicherungsmitteln zum Vermeiden einer Beschädigung des Schlauchs,
- Fig. 6:: ein zweites Ausführungsbeispiel der erfindungsgemässen Klemmvorrichtung, teilweise im Schnitt, und
- Fig. 7:: eine schematische Darstellung eines Fluidsystems mit einer Kombination von einer Pumpvorrichtung mit einer erfindungsgemässen Klemmvorrichtung.

Bei der folgenden Beschreibung beziehen sich relative Lagebezeichnungen wie "oben", "unten", "oberhalb", "unterhalb" usw. auf die Darstellungen in den Figuren, haben jedoch keinen einschränkenden Charakter.

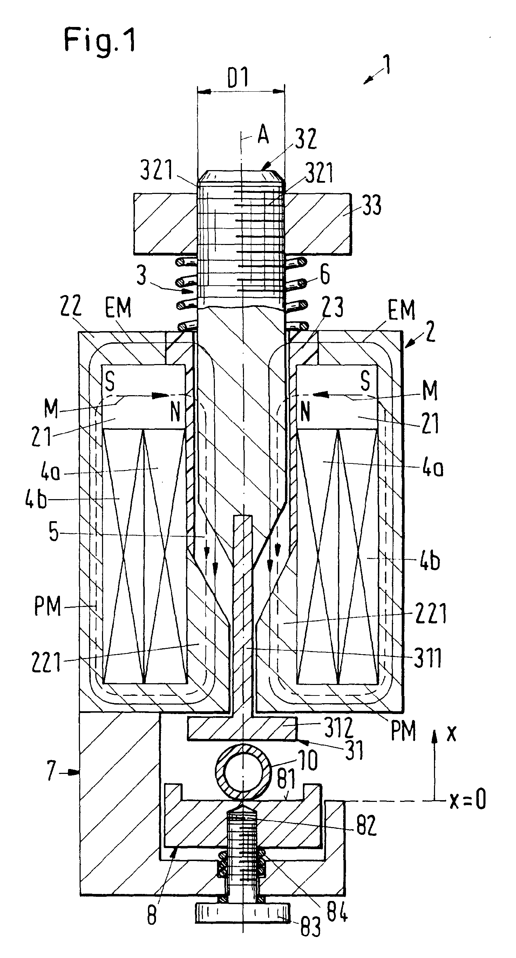

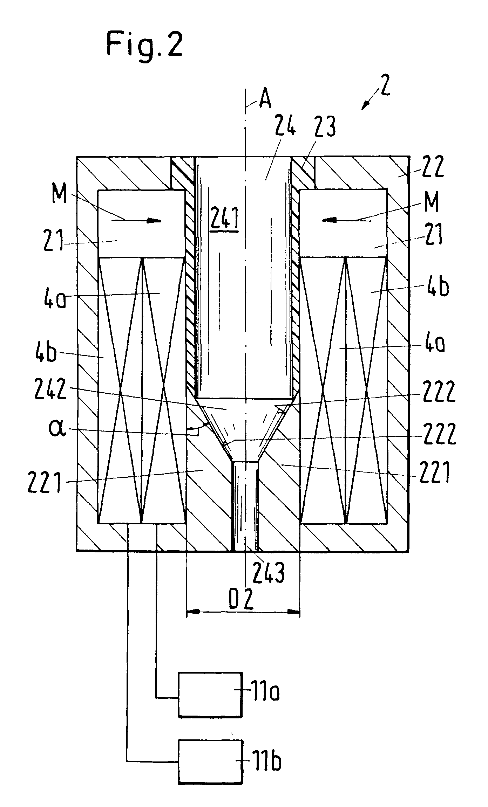

Fig. 1 zeigt in einem Längsschnitt ein erstes Ausführungsbeispiel einer erfindungsgemässen Klemmvorrichtung, die gesamthaft mit dem Bezugszeichen 1 versehen ist. Die Klemmvorrichtung 1 umfasst ein Schliessorgan 3 mit einem Schliessstück 31 zum Abklemmen eines Schlauches 10 und eine permanentmagnetische Halteeinrichtung 2 zum Halten des Schliessorgans 3 in zwei verschiedenen stabilen Gleichgewichtslagen. Zum besseren Verständnis zeigt Fig. 2 in einer zu Fig. 1 analogen Darstellung die permanentmagnetische Halteeinrichtung 2 ohne das Schliessorgan 3.

Die permanentmagnetische Halteeinrichtung 2 (siehe Fig. 1 und Fig. 2) umfasst ein Gehäuse 22, dessen Längsachse mit A bezeichnet ist. Das Gehäuse 22 ist aus einem ferromagnetischen Material gefertigt. Die Halteeinrichtung 2 weist einen zentralen Durchlass 24 zur Aufnahme des Schliessorgans 3 auf. Der Durchlass 24 erstreckt sich in Richtung der Längsachse A und weist ein oberen zylindrischen Bereich 241 auf, an den sich ein konischer Bereich 242 anschliesst, der dann in einen unteren zylindrischen Bereich 243 übergeht, dessen Durchmesser kleiner ist als der Durchmesser des oberen zylindrischen Bereichs 241. Der konische Bereich 242 und der untere zylindrische Bereich 243 werden durch zwei Fortsätze 221 des Gehäuses 22 begrenzt, die sich jeweils in axialer Richtung nach oben erstrecken, und deren oberen Begrenzungsflächen 222 jeweils schräg zur Längsachse A verlaufen, sodass sie den konischen Bereich 242 des Durchlasses 24 bilden. Der halbe Konuswinkel ist mit α bezeichnet. In den oberen zylindrischen Bereich 241 ist eine Hülse 23 eingesetzt, die aus einem nicht-ferromagnetischen Material, beispielsweise Kunststoff hergestellt ist.

In dem Gehäuse 22 der permanentmagnetischen Halteeinrichtung 2 sind zwischen der Hülse 23 und der äusseren Wandung des Gehäuses zwei Permanentmagnete 21 angeordnet, deren Magnetisierung, symbolisch dargestellt durch die Pfeile M, senkrecht zur Längsachse A liegt. In dem hier beschriebenen Ausführungsbeispiel liegt jeweils der Nordpol N innen und der Südpol S aussen, das heisst die Magnetisierung M weist jeweils von aussen nach innen, also zur Längsachse A hin gerichtet. Beispielsweise sind die Permanentmagnete 21 jeweils quaderförmig ausgestaltet und auf beiden Seiten neben dem zentralen Durchlass 24 angeordnet, oder die Permanentmagnete 21 sind jeweils als Halbschalen ausgestaltet, die um den Durchlass 24 herum angeordnet sind.

Unterhalb der Permanentmagnete 21 sind in dem Gehäuse 22 zwei koaxial angeordnete Spulen 4a,4b vorgesehen, welche als Betätigungsmittel dienen, um das Schliessorgan 3 aus seiner Offenstellung in seine Schliessstellung zu bewegen bzw. umgekehrt. Jede der Spulen 4a,4b umgibt den Durchlass 24, wobei die erste Spule 4a bezüglich der radialen Richtung innenliegend angeordnet ist und die zweite Spule 4b aussenliegend um die erste Spule 4a herum angeordnet ist. Jede der Spulen 4a,4b kann also, wenn sie mit Strom gespeist wird, eine in Richtung der Längsachse A gerichtete elektromagnetische Kraft auf das Schliessorgan 3 ausüben. Die an sich bekannten Ansteuer- und Versorgungseinrichtungen 11a, 11b für die Spulen 4a,4b sind in Fig. 2 symbolisch dargestellt. Natürlich sind auch Ausgestaltungen mit nur einer Spule als Betätigungsmittel für das Schliessorgan 3 möglich. Mit zwei separaten Spulen 4a, 4b lässt sich jedoch eine vorteilhafte Fehlertoleranz realisieren, da im Falle eines Fehlers in einer Spule das Schliessorgan 3 noch mittels der anderen Spule betätigt werden kann. Daher sind vorzugsweise auch getrennte Ansteuer- und Versorgungsvorrichtungen 11a, 11b mit getrennten Verstärkern für die beiden Spulen 4a,4b vorgesehen.

Das Schliessorgan 3 (siehe Fig. 1) umfasst einen in der Hülse 23 angeordneten Stab 32, dessen Durchmesser D1 an den Innendurchmesser der Hülse 23 im oberen zylindrischen Teil 241 des Durchlasses 24 angepasst ist, sodass der Stab 32 durch die Hülse 23 geführt ist. Der Stab 32 ist aus einem ferromagnetischen Material gefertigt und verjüngt sich an seinem unteren Ende konisch, wobei der halbe Konuswinkel vorzugsweise gleich gross ist wie der Winkel α, unter dem die Begrenzungsflächen 222 geneigt sind, sodass zwischen dem unteren Ende des Stabs 32 und den Begrenzungsflächen 222 ein Luftspalt 5 existiert, der bezüglich der axialen Richtung von zwei zueinander parallelen Flächen begrenzt wird, die bezüglich der Längsachse A schräg verlaufen. Am unteren Ende des Stabs 32 ist das Schliessstück 31 befestigt, das hier als Stempel mit einem Stiel 311 und einem Kopf 312 ausgebildet ist. Der Stiel 311 erstreckt sich durch den unteren konischen Bereich 243 des Durchlasses 24 hindurch und endet an dem Kopf 312, der ausserhalb des Gehäuses 22 angeordnet ist und dazu dient, den Schlauch 10 abzuklemmen. Das Schliessstück 31 ist aus einem nicht-ferromagnetischen Material hergestellt.

Das obere Ende des Stabs 32 ragt aus dem Gehäuse 22 heraus und ist mit einem Griff 33 versehen, der als mechanisches Betätigungsmittel für das Schliessorgan 3 dient. Der Griff 33 ist auf ein Gewinde 321 am oberen Ende des Stabs 32 aufgeschraubt. Zwischen dem Griff 33 und dem Gehäuse 22 bzw. der Hülse 23 ist ein als Spiralfeder ausgestaltetes Federelement 6 vorgesehen, welches über den Griff 33 auf das Schliessorgan 3 einwirkt und auf dieses eine Federkraft ausübt, die darstellungsgemäss nach oben gerichtet ist. Die Vorspannung des Federelements 6 und damit die von ihm bewirkte Federkraft ist mittels des Griffs 33 einstellbar. Wird der Griff 33 auf dem Gewinde 321 weiter nach unten geschraubt, so wird das Federelement 6 stärker komprimiert, wodurch sich die von ihm ausgeübte Federkraft erhöht. Wird der Griff 33 auf dem Gewinde 321 nach oben geschraubt, so entspannt sich das Federelement, woraus eine Reduzierung der auf das Schliessorgan 3 einwirkenden Federkraft resultiert.

Unterhalb des Gehäuses 22 ist eine an dem Gehäuse 22 befestigte Halterung 7 für ein Gegenstück 8 zum Auflegen des Schlauchs 10 vorgesehen. Das Gegenstück 8 weist eine Auflagefläche 81 für den Schlauch 10 auf und ist so angeordnet, dass der Schlauch 10 zwischen dem Schliessstück 31 des Schliessorgans 3 und dem Gegenstück 8 eingeklemmt werden kann. Das Gegenstück 8 ist bezüglich der axialen Richtung federnd und verstellbar gelagert, um eine Anpassung an den Durchmesser des Schlauchs 10 zu ermöglichen und um die Schliessbewegung zu dämpfen. Dazu ist das Gegenstück 8 mit einer Gewindebohrung 82 versehen, in welche eine Justierschraube 83 eingreift, die sich durch eine gewindelose Bohrung in der Halterung 7 hindurch erstreckt. Zwischen der Unterseite des Gegenstücks 8 und der Halterung 7 ist eine Feder 84 vorgesehen, sodass das Gegenstück 8 bezüglich der Halterung 7 federnd gelagert ist. Durch Drehen der Justierschraube 83 kann das Gegenstück 8 in Richtung der Längsachse A nach oben bzw. nach unten verschoben werden, wodurch die Klemmvorrichtung 1 an den Durchmesser des jeweils verwendeten Schlauchs 10 angepasst werden kann.

Die permanentmagnetische Halteeinrichtung 2 kann, wie noch eingehender erläutert wird, das Schliessorgan 3 in zwei verschiedenen stabilen Gleichgewichtlagen halten, nämlich einer in Fig. 1 dargestellten Offenstellung, in welcher der Schlauch 10 nicht oder nur sehr wenig geklemmt wird, sodass die Flüssigkeit durch den Schlauch 10 hindurchströmen kann, und einer Schliessstellung, in welcher der Schlauch 10 derart zwischen dem Schliessstück 31 und dem Gegenstück 8 eingeklemmt wird, dass keine Flüssigkeit mehr durch den Schlauch 10 hindurchströmen kann. In der Offenstellung (Fig. 1) ist der Strömungsquerschnitt für die Flüssigkeit im Schlauch 10 maximal. Zum Schliessen wird das Schliessorgan 3 in noch zu erläuternder Weise nach unten bewegt und presst dadurch den Schlauch 10 derart zusammen, das der Strömungsquerschnitt Null wird. In dieser Schliessstellung des Schliessorgans 3 ist der Schlauch 10 dann abgeklemmt.

Aus Gründen der besseren Übersicht ist in Fig. 1 das Schliessstück 31 ohne Kontakt mit dem Schlauch 10 dargestellt. In der Praxis ist jedoch der Schlauch 10 üblicherweise auch in der Offenstellung leicht eingeklemmt zwischen dem Schliessstück 31 und dem Gegenstück 8.

Ein wesentliches Merkmal der Erfindung ist es, dass die permanentmagnetische Halteeinrichtung 2 das Schliessorgan 3 in beiden Gleichgewichtslagen (Offenstellung und Schliessstellung) halten kann. Während des Haltens benötigt die Klemmvorrichtung 1 keine Energie in Form von Strom, weshalb die Klemmvorrichtung 1 enorm sparsam bezüglich des Energieverbrauchs ist.

In Fig. 1 ist der von den Permanentmagneten 21 generierte permanentmagnetische Fluss durch die beiden gestrichelt gezeichneten Feldlinien PM symbolisch dargestellt. Der permanentmagnetische Fluss PM fliesst vom Nordpol N des Permanentmagneten durch die Hülse 23 in den ferromagnetischen Stab 32, wird von diesem in axialer Richtung nach unten geführt, fliesst durch den Luftspalt 5 in den Fortsatz 221 des Gehäuses 22, und wird dann vom Gehäuse 22 zunächst in radialer Richtung nach aussen und dann in axialer Richtung nach oben zum Südpol S des Permanentmagneten 21 zurückgeführt.

Ebenfalls eingezeichnet in Fig. 1 sind zwei Feldlinien EM (durchgezogene Linien) des elektromagnetischen Flusses, der von den Spulen 4a,4b generiert wird, aber nur vorhanden ist, wenn das Schliessorgan aus der Offen- in die Schliessstellung oder aus der Schliessstellung in die Offenstellung geschaltet wird. Diese Schaltvorgänge werden weiter hinten erläutert.

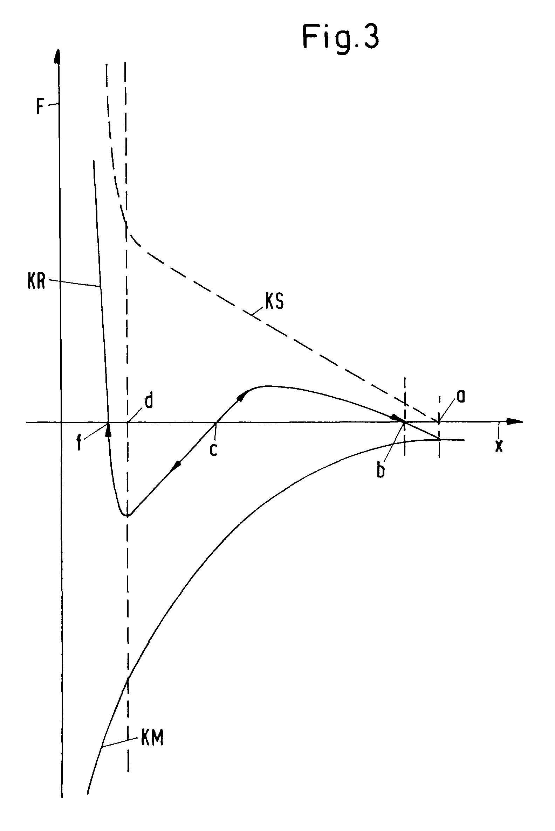

Im Folgenden wird nun das Halten des Schliessorgans 3 in den beiden Gleichgewichtslagen anhand von Fig. 3 näher erläutert. Ohne Beschränkung der Allgemeinheit werden Kräfte, die nach unten wirken, also in Richtung auf die Schliessstellung, als negativ dargestellt und bezeichnet, und Kräfte die nach oben wirken als positiv. Während des Haltens in der Offen- oder der Schliessstellung sind die Spulen 4a,4b deaktiviert, sodass sie keine elektromagnetische Kraft auf das Schliessorgan 3 ausüben. Dann wirken auf das Schliessorgan 3 im wesentlichen die folgenden Kräfte: Die negative magnetische Kraft, welche von den Permanentmagneten 21 ausgeübt wird, eine positive Kraft, welche der Schlauch 10 bewirkt und welche im Folgenden als Schlauchkraft bezeichnet wird, sowie die positive Federkraft, welche das Federelement 6 bewirkt. Der Betrag dieser Kräfte ist von einer Wegkoordinate x abhängig. Die Wegkoordinate x (siehe Fig. 1) gibt den Abstand zwischen der Auflagefläche 81 des Gegenstücks 8 und der ihr zugewandten Begrenzungsfläche des Kopfs 312 des Schliessstücks 31 an, wobei x=0 bedeutet, dass der Kopf 312 des Schliessstücks 31 auf der Auflagefläche 81 des Gegenstücks 8 aufliegt.

In Fig. 3 sind verschiedenen Kraft-Weg-Kennlinien für den Fall aufgetragen, dass die Spulen 4a, 4b deaktiviert sind, also nicht mit Strom gespeist werden. Die Wegkoordinate x nimmt nach rechts zu und der Betrag der jeweiligen Kraft F nach oben für positive Kräfte und nach unten für negative Kräfte.

Die Kennlinie KS repräsentiert die Schlauchkraft. Für x=a (oder x>a) ist der Schlauch ganz offen, die Schlauchkraft ist Null. Mit kleiner werdendem x nimmt die Schlauchkraft im Bereich d<x<a zunächst näherungsweise linear zu. Dieser Bereich entspricht dem "Kollabieren" des Schlauchs, das heisst der Schlauch wird immer mehr zusammengedrückt, wodurch sich der offene Strömungsquerschnitt in ihm reduziert. Bei x=d ist der Schlauch vollständig "kollabiert", das heisst er ist so eingeklemmt, dass der offene Strömungsquerschnitt in ihm Null ist. Bei weiterer Reduzierung von x (f<x<d) wird die Schlauchwand komprimiert, was zu einem sehr steilen Anstieg in der Schlauchkraft führt.

Die magnetische Kennlinie KM gibt die Abhängigkeit der permanentmagnetischen Kraft auf das Schliessorgan 3 in Abhängigkeit von x wieder. Mit kleiner werdendem x wird auch der Luftspalt 5 zwischen dem Stab 32 und den Fortsätzen 221 des Gehäuses 22 kleiner, woraus eine Erhöhung des permanentmagnetischen Flusses und damit der von ihm bewirkten magnetischen Kraft auf das Schliessorgan 3 resultiert. Vorzugsweise ist der Spalt 5 so bemessen, dass er für x=0 gerade ganz geschlossen ist. Da die magnetische Kraft im wesentlich proportional zum Quadrat des magnetischen Flusses ist, steigt der Betrag der magnetischen Kraft mit kleiner werdendem x im wesentlichen quadratisch an. Diese Abhängigkeit führt qualitativ zu der in Fig. 3 dargestellten Kennlinie KM.

Für die positive Federkraft, welche das Federelement 6 bewirkt, ist in Fig. 3 keine gesonderte Kennlinie eingezeichnet. Die Federkraft hängt im wesentlichen linear vom Weg x ab, wobei der Betrag der Federkraft mit kleiner werdendem x grösser wird. Rein qualitativ verläuft die Kennlinie für die Federkraft also in gleicher Weise wie die Kennlinie KS der Schlauchkraft in ihrem linearen Bereich (d<x<a). Da es für das Verständnis ausreichend ist, wird die Kennlinie KS im Folgenden als repräsentativ für die Summe aus der Schlauchkraft und der von dem Federelement 6 bewirkten Federkraft angesehen. Für solche Ausgestaltungen, bei denen kein Federelement 6 vorgesehen ist, repräsentiert die Kennlinie KS also die Abhängigkeit der Schlauchkraft von der Wegkoordinate x, und für solche bevorzugten Ausgestaltungen wie der hier beschriebenen, bei denen das Federelement 6 vorhanden ist, repräsentiert die Kennlinie KS die Abhängigkeit der Summe aus der Schlauchkraft und der Federkraft von der Wegkoordinate x. Da die Schlauchkraft in ihrem linearen Bereich eine Wegabhängigkeit aufweist, welche der einer Feder entspricht, der Schlauch sich also etwa wie eine Feder verhält, wird im Folgenden die Summe aus der Schlauchkraft und der von dem Federelement 6 bewirkten Kraft der Einfachheit halber als Federkraft bezeichnet. Diese beinhaltet sowohl die Kraft der "Schlauchfeder" als auch die des Federelements 6.

Die Kennlinie für die resultierende Gesamtkraft, welche auf das Schliessorgan 3 einwirkt, ergibt sich durch Addition der magnetischen Kennlinie KM und der Kennlinie KS. Die Kennlinie für die Gesamtkraft ist in Fig. 3 mit dem Bezugszeichen KR bezeichnet. Wie dies Fig. 3 zeigt, hat die Kennlinie KR drei Nulldurchgänge, nämlich bei den Werten x=b, x=f und x=c. Bei diesen Nulldurchgängen ist die resultierende Gesamtkraft auf das Schliessorgan 31 Null, das heisst für diese drei Werte b, f, c befindet sich das Schliessorgan im Kräftegleichgewicht. Zwei der Gleichgewichtslagen, nämlich x=f und x=b, sind stabile Gleichgewichtslagen. Befindet sich beispielsweise das Schliessorgan 3 in der Position x=f und wird es in Richtung kleiner werdender x-Koordinate aus dieser Lage ausgelenkt, so überwiegt die positive Federkraft und bewirkt wieder eine Vergrösserung von x, das heisst, das Schliessorgan 3 kehrt in die Gleichgewichtslage x=f zurück. Lenkt man das Schliessorgan 3 aus der Gleichgewichtslage x=f in Richtung grösser werdender x-Koordinate aus, so überwiegt die negative magnetische Kraft und zieht das Schliessorgan 3 in die Gleichgewichtslage x=f zurück. Folglich ist x=f eine stabile Gleichgewichtslage. In analoger Weise ergibt sich, dass auch der Wert x=b einer stabilen Gleichgewichtslage des Schliessorgans 3 entspricht. Die stabile Gleichgewichtslage x=f ist die Schliessstellung des Schliessorgans 3 und die stabile Gleichgewichtslage x=b seine Offenstellung.

Die dritte Gleichgewichtslage bei x=c ist eine labile Gleichgewichtslage. Befindet sich das Schliessorgan 3 in der Position x=c und lenkt man es nur geringfügig in Richtung grösser werdender x-Koordinate aus dieser Gleichgewichtslage aus, so überwiegt die Federkraft, wodurch x noch weiter vergrössert wird, bis das Schliessorgan 3 bei x=b eine stabile Gleichgewichtslage (Offenstellung) einnimmt. Lenkt man das Schliessorgan 3 aus der Position x=c in Richtung kleiner werdender x-Koordinate aus, so überwiegt die negative magnetische Kraft, wodurch x noch weiter verkleinert wird, bis das Schliessorgan bei x=f die andere stabile Gleichgewichtslage (Schliessstellung) einnimmt.

Somit existieren genau zwei stabile Gleichgewichtslagen, nämlich die Offenstellung und die Schliessstellung des Schliessorgans 3, in denen sich die von den Permanentmagneten 21 auf das Schliessorgan 3 ausgeübte Kraft und die auf das Schliessorgan 3 einwirkende Federkraft gegenseitig dauerhaft kompensieren. Die permanentmagnetische Halteeinrichtung 2 ist ein bistabiles, passives Haltesystem, wobei passiv in dem Sinne zu verstehen ist, dass dem Haltesystem keine Energie, z. B. in Form von Strom, zugeführt werden muss, um das Schliessorgan in der Offen- oder in der Schliessstellung zu halten.

Um die beiden Kennlinien KM und KS so aufeinander abzustimmen, dass sich die gewünschte resultierende Kennlinie KS ergibt, stehen verschiedene Massnahmen zur Verfügung, von denen hier nur einige in nicht abschliessender Aufzählung erwähnt werden.

Die Kennlinie KS kann über die Materialeigenschaften des Materials, aus welchem der Schlauch 10 hergestellt ist, sowie durch die Geometrie des Schlauchs 10, beispielsweise die Dicke der Schlauchwand, verändert werden. Ferner kann die Kennlinie KS durch die Wahl des Federelements 6 variiert werden, oder über die Vorspannung des Federelements 6, die mittels des Griffs 33 (Fig. 1) einstellbar ist.

Die Änderung bzw. Anpassung der Kennlinie KM für die von den Permanentmagneten 21 verursachte magnetische Kraft kann erfolgen über: Material, Grösse, Form und Stärke der Permanentmagneten 21, Materialeigenschaften wie die Sättigungsmagnetisierung, sowie Geometrie derjenigen Bauteile, welche den permanentmagnetischen Fluss führen, also z. B. der Stab 32, die Fortsätze 221 und das Gehäuse 22. So lässt sich die permanentmagnetische Kraft beispielsweise über den Aussendurchmesser D1 des Stabs 32 (siehe Fig. 1) verändern oder über die Ausdehnung D2 (siehe Fig. 2) der Fortsätze 221 in radialer Richtung.

Insbesondere kann der Verlauf der Kennlinie KM der magnetischen Kraft über den Winkel α (Fig. 2) geändert werden, welcher die Neigung der Begrenzungsflächen 222 der Fortsätze 221 bestimmt. Aus fertigungstechnischen Gründen wird α vorzugsweise zwischen 0° und 90° gewählt. Kleine Werte von α haben zur Folge, dass die magnetische Kennlinie KM flacher, also mit geringerer Steigung insbesondere für kleine Werte von x, verläuft, während grössere Werte von α die magnetische Kennlinie KM steiler werden lassen. Für α = 90° verlaufen die Begrenzungsflächen 222 senkrecht zur Längsachse A, das heisst der konische Bereich 242 des Durchlasses 24 (Fig. 2) ist nicht vorhanden.

Durch diese Massnahmen ist es möglich, die beiden Kennlininen, nämlich die Kennlinie KM für die magnetische Kraft und die Kennlinie KS für die Summe aus der von dem Schlauch 10 und von dem Federelment 6 verursachten Federkraft, so aufeinander abzustimmen, dass für das Schliessorgan 3 zwei stabile Gleichgewichtslagen existieren, wenn kein Strom in die Spulen 4a,4b eingespeist wird.

Es wird nun erläutert, wie das Schliessorgan 3 aus einer stabilen Gleichgewichtslage in die andere stabile Gleichgewichtslage gebracht wird. Zunächst wird davon ausgegangen, dass sich das Schliessorgan in seiner in Fig. 1 veranschaulichten Offenstellung befindet und in die Schliessstellung gebracht werden soll. Dazu sind bei dem hier beschriebenen Ausführungsbeispiel die beiden Spulen 4a,4b (siehe Fig 1, Fig. 2) als Betätigungsmittel vorgesehen. Wie bereits erläutert, sind die Spulen 4a, 4b so angeordnet, dass sie bei ihrer Aktivierung durch Beaufschlagung mit Strom auf das Schliessorgan 3 eine in Richtung auf die Schliessstellung oder in Richtung auf die Offenstellung wirkende elektromagnetische Kraft ausüben. Die Richtung der elektromagnetischen Kraft hängt dabei von der Polarität des Stromes ab, der in die Spulen 4a,4b eingespeist wird. Ohne Beschränkung der Allgemeinheit sei vereinbart, dass ein Strom mit positivem Vorzeichen eine in Richtung der Längsachse A nach unten - also auf die Schliessstellung hin - gerichtete elektromagnetische Kraft auf das Schliessorgan 3 bewirkt und ein Strom mit negativem Vorzeichen eine in Richtung der Längsachse A nach oben gerichte elektomagnetische Kraft. In Fig. 1 ist der elektromagnetische Fluss, der aus einem positiven Strom resultiert, symbolisch durch die beiden Feldlinien EM veranschaulicht.

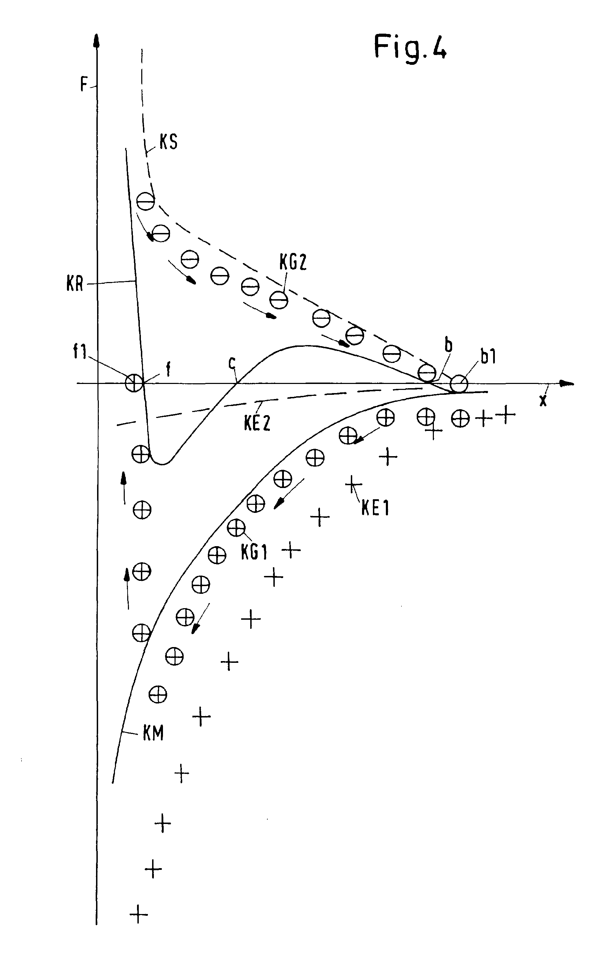

Fig. 4 zeigt in einer zu Fig. 3 analogen Darstellung mehrere Kraft-Weg-Kennlinien. KS bezeichnet wiederum die Kennlinie für die Federkraft, welche aus dem Schlauch 10 und dem Federelement 6 resultiert, KM bezeichnet die Kennlinie der magnetischen Kraft, die von den Permanentmagneten 21 verursacht wird und KR die Kennlinie der resultierenden Gesamtkraft. Die Kennlinien KM, KR beziehen sich auf den Fall, dass die Spulen 4a,4b nicht mit Strom beaufschlagt werden. In diesem Fall existieren für das Schliessorgan 3 die beiden stabilenen Gleichgewichtslagen bei x=b (Offenstellung) und x=f (Schliessstellung).

Werden nun die Spulen 4a,4b mit einem positiven Strom beaufschlagt, so verursachen sie einen elektromagnetischen Fluss, der eine negative elektromagnetische Kraft auf das Schliessorgan 3 ausübt. Für einen positiven Strom haben der elektromagnetische Fluss und der permanentmagnetische Fluss die gleiche Richtung, das heisst sie addieren sich in ihrer Wirkung. Die gesamte magnetische Kraft, also die Summe aus der permanentmagnetischen Kraft und der elektromagnetischen Kraft, ist proportional zum Quadrat der Summe aus dem elektromagnetischen und dem permanentmagnetischen Fluss. Diese gesamte magnetische Kraft, bzw. ihre Abhängigkeit von x, ist in Fig. 4 durch die Kennlinie KE1 veranschaulicht, welche mit den Symbolen + eingezeichnet ist. Das Symbol + soll dabei anzeigen, dass der Strom für die Spulen 4a,4b positives Vorzeichen hat. Die Kennlinie KG1, welche die Gesamtkraft repräsentiert, die bei positivem Strom durch die Spulen 4a,4b auf das Schliessorgan 3 einwirkt, ergibt sich durch Addition der Kennlinien KS und KE1. Diese mit KG1 bezeichnete Kennlinie ist mit den Symbolen ⊕ dargestellt. Wie dies Fig. 4 zeigt, weist die Kennlinie KG1 nur noch einen Nulldurchgang bei x=f1 auf, wobei f1<f ist. Dieser Nulldurchgang bei f1 ist eine stabile Gleichgewichtslage. Für alle Werte von x, die grösser als f1 sind, ist die gesamte magnetische Kraft (Kennlinie KE1) betragsmässig stets grösser als die Federkraft (Kennlinie KS), sodass die resultierende Gesamtkraft (Kennlinie KG1) stets negativ ist. Befindet sich also das Schliessorgan anfänglich in seiner Offenstellung (x=b) und wird dann ein positiver Strom in die Spulen 4a,4b eingespeist, so bewegt sich das Schliessorgan in die Gleichgewichtslage f1. Nach Abschalten des Stroms durch die Spulen 4a,4b nimmt das Schliessorgan 3 die stabile Gleichgewichtslage x=f auf der Kennlinie KR ein und befindet sich somit in seiner Schliessstellung. Der Schlauch 10 ist abgeklemmt, das heisst er ist derart zwischen dem Schliessstück 31 und dem Gegenstück 8 eingeklemmt, dass keine Flüssigkeit mehr durch ihn hindurchströmen kann.

Um das Schliessorgan 3 aus der Offen- in die Schliessstellung zu bewegen, ist es nicht notwendig, den positiven Strom solange eingeschaltet zu lassen, bis das Schliessorgan 3 die Gleichgewichtslage bei x=f1 angenommen hat. Der Strom durch die Spulen 4a,4b kann bereits abgeschaltet werden, sobald sich das Schliessorgan 3 bei einer Wegkoordinate x befindet, die kleiner ist als c. Auf der Kennlinie KR, die den stromlosen Fall repräsentiert, befindet man sich dann nämlich bereits links der labilen Gleichgewichtslage x=c, sodass das Schliessorgan 3 selbsttätig, das heisst ohne Unterstützung durch die elektromagnetische Kraft bzw. den elektromagnetischen Fluss, seine stabile Gleichgewichtslage bei x=f (Schliessstellung) einnimmt.

Um das Schliessorgan 3 aus der Schliessstellung in die Offenstellung zu bewegen, werden die Spulen 4a,4b mit einem negativen Strom beaufschlagt. In diesem Fall ist der von den aktivierten Spulen 4a,4b generierte elektromagnetische Fluss entgegengesetzt gerichtet zum permanentmagnetischen Fluss, das heisst diese beiden Flüsse schwächen sich in ihrer Wirkung ab. Die gesamte magnetische Kraft, die von den Permanentmagneten 21 und den aktivierten Spulen 4a,4b auf das Schliessorgan 3 ausgeübt wird, ist also für einen negativen Strom proportional zum Quadrat der Differenz aus dem Betrag des permanentmagnetischen Flusses und dem Betrag des elektromagnetischen Flusses. Die Wegabhängigkeit dieser gesamten magnetischen Kraft für den Fall eines negativen Stroms in den Spulen 4a,4b ist in Fig. 4 durch die Kennlinie KE2 wiedergegeben, die mit den Symbolen - dargestellt ist. Das Symbol - soll dabei anzeigen, dass der Strom für die Spulen 4a,4b negatives Vorzeichen hat. Die Kennlinie KG2, welche die Gesamtkraft repräsentiert, die bei negativem Strom durch die Spulen 4a,4b auf das Schliessorgan 3 einwirkt, ergibt sich durch Addition der Kennlinien KS und KE2. Diese mit KG2 bezeichnete Kennlinie ist mit den Symbolen e dargestellt. Wie dies Fig. 4 zeigt, weist die Kennlinie KG2 nur noch eine Nullstelle bei x=b1 auf, wobei b1>b ist. Diese Nullstelle bei b1 ist eine stabile Gleichgewichtslage. Für alle Werte von x, die kleiner als b1 sind, ist die gesamte magnetische Kraft (Kennlinie KE2) betragsmässig stets kleiner als die Federkraft (Kennlinie KS), sodass die resultierende Gesamtkraft (Kennlinie KG2) stets positiv ist. Befindet sich also das Schliessorgan 3 anfänglich (das heisst vor Aktivieren der Spulen 4a,4b) in seiner Schliessstellung (x=f) und wird dann ein negativer Strom in die Spulen 4a, 4b eingespeist, so bewegt sich das Schliessorgan 3 in die Gleichgewichtslage b1. Nach Abschalten des Stroms durch die Spulen 4a,4b nimmt das Schliessorgan 3 die stabile Gleichgewichtslage x=b auf der Kennlinie KR ein und befindet sich somit in seiner Offenstellung.

Analog wie vorne beschrieben, reicht es auch für den Vorgang des Öffnens aus, wenn der negative Strom in den Spulen 4a,4b solange eingeschaltet bleibt, bis sich das Schliessorgan 3 in einer Position mit x>c befindet. Wird dann der Strom abgeschaltet, bewegt sich das Schliessorgan 3 weiter, bis es seine stabile Gleichgewichtslage bei x=b eingenommen hat, weil es sich bereits rechts der labilen Gleichgewichtslage x=c für den stromlosen Fall befindet.

Mittels der Ansteuer- und Versorgungseinrichtungen 11a, 11b für die Spulen 4a,4b kann der positive oder negative Strom in einfacher Weise so eingestellt werden, dass zumindest qualitativ die in Fig. 4 gezeigten Kennlinien KE1 und KE2 resultieren. Die für das Öffnen massgebliche Kennlinie KE2 wird vorzugsweise so eingestellt, dass sie möglichst nahe unterhalb der x-Achse verläuft. In der Praxis ist es normalerweise nicht möglich, die Kennlinie KE2 derart einzustellen, dass sie genau auf der x-Achse liegt.

Wie vorangehend beschrieben, lässt sich also das Schliessorgan durch Beaufschlagen der Spulen 4a,4b mit einem positiven oder mit einem negativen Strom aus der Offen- in die Schliessstellung oder aus der Schliessin die Offenstellung bewegen. Dazu werden die Richtung (das Vorzeichen) und der Betrag des Stroms jeweils so eingestellt, dass für das Schliessorgan 3 nur noch eine stabile Gleichgewichtslage (offen oder zu) existiert, solange der Strom in den Spulen 4a,4b fliesst.

Wie bereits erwähnt, ist es keinesfalls notwendig zwei separate Spulen 4a,4b vorzusehen. Das hier beschriebene Ausführungsbeispiel funktioniert in genau gleicher Weise auch dann, wenn nur eine Spule vorgesehen ist. Die Ausführung mit zwei Spulen hat jedoch den Vorteil, fehlertolerant zu sein. Im normalen Betrieb wird das Schalten zwischen Offen- und Schliessstellung des Schliessorgans 3 durch Beaufschlagung beider Spulen 4a,4b mit Strom bewirkt. Falls nun eine Spule 4a oder 4b (oder eine der Versorgungs- und Ansteuervorrichtungen 11a, 11b) ausfällt, z. B. aufgrund des Bruchs eines elektrischen Leiters, so bleibt die Klemmvorrichtung 1 vollständig betriebsfähig, weil das Schalten in die Offen- bzw. Schliessstellung noch mit der anderen Spule 4b oder 4a bewirkt werden kann. Falls nur eine der beiden Spulen 4a oder 4b für das Schalten verwendet wird, muss natürlich in dieser Spule ein entsprechend grösserer Strom fliessen, um im wesentlichen einen gleich grossen elektromagnetischen Fluss zu generieren wie im Falle, dass beide Spulen zum Generieren des elektromagnetischen Flusses verwendet werden.

Die Klemmvorrichtung 1 kann ferner in einfacher Weise mechanisch betätigt werden, beispielsweise wenn beide Spulen 4a,4b oder die gesamte Stromversorgung ausfallen. Dazu ist der Griff 33 (siehe Fig. 1) vorgesehen. Befindet sich das Schliessorgan 3 in der Offenstellung, so kann es durch Ausüben einer nach unten gerichteten (negativen) Kraft, beispielsweise durch einen Druck oder Schlag per Hand, in die Schliessstellung gebracht werden. Diese Kraft muss lediglich ausreichen, um das Schliessorgan 3 in eine Position mit x<c (siehe Fig. 3) zu bewegen. Dann sorgt die permanentmagnetische Halteeinrichtung 2 selbständig dafür, dass das Schliessorgan 3 seine Schliessstellung bei x=f einnimmt und beibehält. Umgekehrt kann das Schliesselement 3 in sinngemäss gleicher Weise durch Ausüben einer nach oben gerichteten (positiven) Kraft auf den Griff 33, beispielsweise durch Ziehen per Hand, aus der Schliessstellung in die Offenstellung gebracht werden.

Falls die Klemmvorrichtung elektromagnetisch betätigt wird, ist eine Schaltzeit von weniger als 100 Millisekunden, beispielsweise etwa 80 Millisekunden, problemlos realisierbar. Mit der Schaltzeit ist die Zeit gemeint, welche das Schliesselement 3 benötigt, um sich aus der einen Gleichgewichtslage in die andere Gleichgewichtslage zu bewegen.

Eine weitere vorteilhafte Massnahme besteht darin, Sicherungsmittel vorzusehen, welche so ausgestaltet sind, dass der Abstand zwischen dem Gegenstück 8 und dem mit ihm zusammenwirkenden Schliessstück 31 des Schliessorgans 3 stets grösser ist als ein Minimalwert. Durch diese Massnahme lässt es sich insbesondere beim Schliessvorgang, bei welchem sich das Schliessstück 31 abwärts bewegt und den Schlauch 10 gegen das Gegenstück 8 presst, verhindern, dass der Schlauch 10 beschädigt oder durchtrennt wird. Zusätzlich erfolgt eine Dämpfung durch die Feder 84.

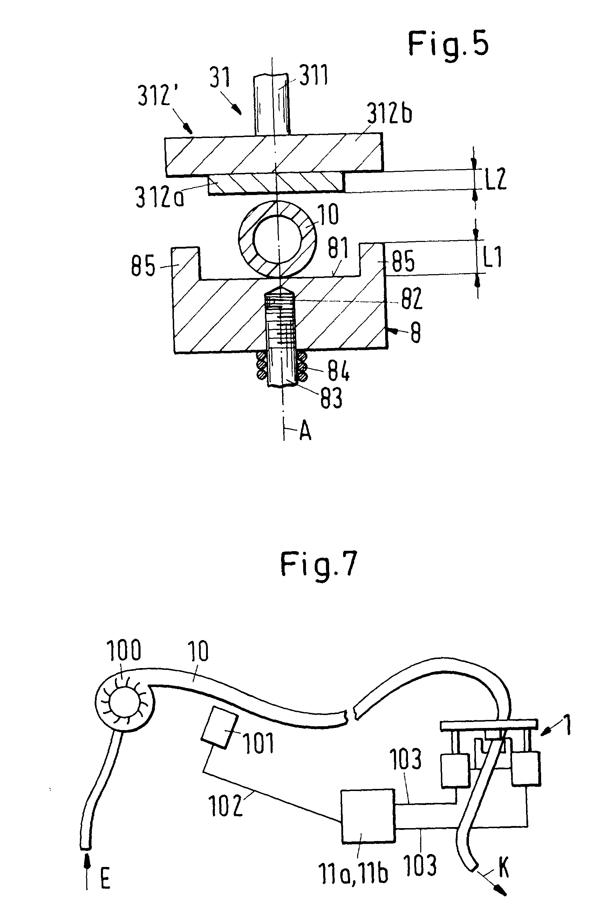

Fig. 5 veranschaulicht eine mögliche Ausgestaltung solcher Sicherungsmittel. Das Gegenstück 8 weist zwei Vorsprünge 85 auf, die in axialer Richtung jeweils um einen Betrag 11 über die Auflagefläche 81 für den Schlauch 10 hinausragen. Der Kopf 312' des Schliessstücks 31 des Schliessorgans 3 umfasst zwei scheibenförmige Elemente 312a und 312b, die bezüglich der Längsachse A übereinander angeordnet sind. Das untere, näher am Gegestück 8 befindliche Element 312a hat eine axiale Höhe 12, womit seine Ausdehnung in Richtung der Längsachse A gemeint ist, die kleiner ist als der Betrag 11, um den die Vorsprünge 85 über die Auflagefläche 81 des Gegenstücks 8 hinausragen. In radialer Richtung ist das untere Element 312a so bemessen, dass es zwischen die beiden Vorsprünge 85 passt. Das obere Element 312b, das weiter von dem Gegenstück 8 entfernt ist, ist dagegen in radialer Richtung so bemessen, dass es nicht zwischen die Vorsprünge 85 passt.

Wird nun das Schliessorgan 3 und damit das Schliessteil 31 nach unten bewegt, so bewegt es sich höchstens soweit, bis das obere Element 312b auf den beiden Vorsprüngen 85 aufliegt. Dies ist der minimal mögliche Abstand zwischen dem Schliessstück 31 und der Auflagefläche 81 des Gegenstücks 8. Dieser Minimalwert beträgt I1-I2. Somit steht für den Schlauch 10 stets ein Spalt zwischen dem unteren Element 312 a des Schliessstücks 31 und der Auflagefläche 81 zur Verfügung, der mindestens die axiale Höhe 11-12 hat. Es versteht sich, dass I1 und I2 so bemessen werden, bzw. so an den jeweils verwendeten Schlauch angepasst werden, dass einerseits I1-I2nicht grösser ist als d (siehe Fig. 3), also der Wert von x, bei welchem der Strömungsquerschnitt im Schlauch 10 vollständig geschlossen ist, und andererseits ein Durchtrennen des Schlauchs 10 beim Schliessen der Klemmvorrichtung 1 sicher verhindert wird. In der Praxis hat es sich insbesondere bewährt, wenn die Differenz 11-12 ungefähr das 1,2-fache bis 1,8-fache der Wandstärke des Schlauchs 10 beträgt.

Als zusätzliche Sicherheitsmassnahme ist das Gegenstück 8 wie vorne erläutert bezüglich der axialen Richtung mittels der Feder 84 federnd gelagert, sodass das Schliessen bzw. das Abklemmen des Schlauches 10 gedämpft erfolgt.

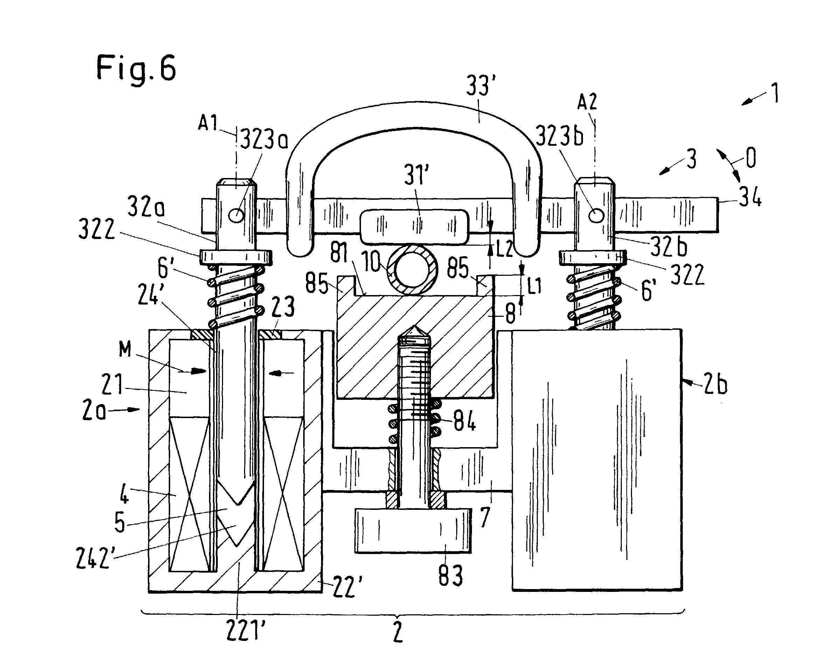

Fig. 6 zeigt, teilweise im Schnitt, ein zweites Ausführungsbeispiel der erfindungsgemässen Klemmvorrichtung 1, das sich in der Praxis besonders bewährt hat. Die prinzipielle Funktionsweise ist identisch mit der des ersten Ausführungsbeispiels und wird daher nicht mehr erläutert. Von der Funktion her identische oder gleichwertige Teile sind mit den gleichen, bereits erläuterten Bezugszeichen versehen wie bei dem ersten Ausführungsbeispiel, wobei teilweise zusätzlich ein Strich' an den Bezugszeichen verwendet wird, um auf die unterschiedliche Ausgestaltung hinzuweisen. Im Folgenden werden die Unterschiede zum ersten Ausführungsbeispiel beschrieben, ansonsten gelten die Erläuterungen bezüglich des ersten Ausführungsbeispiels in sinngemäss gleicher Weise auch für das zweite Ausführungsbeispiel.

Bei dem zweiten Ausführungsbeispiel gemäss Fig. 6 umfasst das Schliessorgan 3, welches in Fig. 6 in seiner Offenstellung dargestellt ist, zwei stabförmige Schenkel 32a, 32b sowie eine Querstange 34, an welcher die Schenkel 32a, 32b beabstandet und parallel zueinander befestigt sind. Die beiden Schenkel 32a,32b erstrecken sich jeweils im wesentlichen senkrecht zur Querstange 34. Das Schliessstück 31' ist an der Querstange 34 zwischen den beiden Schenkeln 32a, 32b angeordnet, kann aber auch durch die Querstange 34 selbst realisiert sein. Die permanentmagnetische Halteeinrichtung 2 umfasst zwei permanentmagnetische Halter 2a,2b, von denen einer im Schnitt dargestellt ist. Beide Halter 2a,2b sind gleich ausgestaltet und nehmen jeweils einen der beiden Schenkel 32a, 32b auf, das heisst der Schenkel 32a wird durch den Halter 2a gehalten und der Schenkel 32b durch den Halter 2b.

Bezüglich des Haltens des Schliessorgans 3 in den beiden stabilen Gleichgewichtslagen (Offen- und Schliessstellung) haben die beiden Schenkel 32a, 32b und die sie jeweils umgebenden permanentmagnetischen Halter 2a,2b die sinngemäss gleiche Funktion wie der Stab 32 und die permanentmagnetische Halteeinrichtung 2 des ersten Ausführungsbeispiels. Da jedoch bei dem zweiten Ausführungsbeispiel das Schliessstück 31' an der Querstange 34 angeordnet ist, ist bei jedem permanentmagnetischen Halter 2a,2b anstelle des Durchlasses 24 aus Fig. 1 eine Ausnehmung 24' vorgesehen, die sich jeweils in Richtung der Längsachse A1 bzw. A2 des permanentmagnetischen Halters 2a bzw. 2b erstreckt und mit einem konischen Bereich 242' im Innern des Gehäuses 22' endet. Dieses Ende wird von dem Fortsatz 221' des Gehäuses 22' gebildet, welcher den beiden Fortsätzen 221 in Fig. 2 entspricht. Das in der Ausnehmung 24' angeordnete Ende des Schenkels 32a bzw. 32b ist jeweils konisch ausgestaltet, in sinngemäss gleicher Weise wie bei dem Stab 32 des ersten Ausführungsbeispiels.

In Fig. 6 ist in dem permanentmagnetischen Halter 2a nur eine Spule 4 zum Erzeugen der elektromagnetischen Kraft für den Wechsel zwischen den beiden stabilen Gleichgewichtslagen dargestellt. Natürlich können auch bei dem zweiten Ausführungsbeispiel in jedem Halter 2a,2b in sinngemäss gleicher Weise wie vorne beschrieben jeweils zwei separate Spulen mit getrennten Ansteuer- und Versorgungsvorrichtungen 11a, 11b vorgesehen sein, um eine vorteilhafte Fehlertoleranz zu realisieren.

Für jeden Schenkel 32a,32b ist ein Federelement 6' vorgesehen, um eine nach oben, also in Richtung der Offenstellung, gerichtete Federkraft auf das Schliessorgan 3 auszuüben. An dem aus dem Gehäuse 22' herausragenden Teil jedes Schenkels 32a,32b ist jeweils ein Teller 322 vorgesehen, auf welchem sich das Federelement 6' abstützt. Mit seinem anderen Ende liegt das Federelement 6' auf dem Gehäuse 22' bzw. auf der Hülse 23 auf, welche in die Ausnehmung 24' eingepasst ist. Die Funktion der Federelemente 6' entspricht derjenigen des Federelements 6 in Fig. 1. Die Federelemente 6' bewirken eine Federkraft auf das Schliessorgan 3, welche der von den Permanentmagneten 21 bewirkten Kraft entgegenwirkt.

Die Halterung 7 für das Gegenstück 8 ist zwischen den beiden permanentmagnetischen Haltern 2a,2b angeordnet, sodass das Gegenstück 8 mit seiner Auflagefläche 81 dem Schliessstück 31' gegenüberliegt. In gleicher Weise wie beim ersten Ausführungsbeispiel ist das Gegenstück 8 mittels der Justierschraube 83 bezüglich der Richtung der parallelen Längsachsen A1,A2 verstellbar und mittels der Feder 84 federnd in der Halterung 7 gelagert.

Um das Einlegen des Schlauchs 10 in die Klemmvorrichtung 1 zu vereinfachen, sind die Schenkel 32a,32b jeweils mittels Stiften 323a,323b mit der Querstange 34 verbunden. Nach Entfernen des Stifts 323b kann die Querstange 34 um den Stift 323a herum nach oben geschwenkt werden, wie dies der Pfeil O andeutet, sodass der Schlauch 10 auf die Auflagefläche 81 des Gegenstücks 8 aufgelegt werden kann. Danach wird die Querstange 34 nach unten geschwenkt und der Stift 323b eingesetzt.

Als mechanisches Betätigungsmittel ist an der Querstange der Griff 33' angebracht. Durch Ausüben einer nach unten gerichteten Kraft auf den Griff 33', beispielsweise durch Drücken per Hand, kann das Schliessorgan aus der Offenstellung in die Schliessstellung gebracht werden. Umgekehrt kann durch Ausüben einer nach oben gerichteten Kraft auf den Griff 33', beispielsweise durch Ziehen per Hand, das Schliessorgan 3 aus der Schliessstellung in die Offenstellung gebracht werden.

Als Sicherungsmittel zum Vermeiden einer Verletzung oder eines Durchtrennen des Schlauchs 10, sind auch bei dem zweiten Ausführungsbeispiel an dem Gegenstück 8 die beiden Vorsprünge 85 vorgesehen, die in axialer Richtung jeweils um einen Betrag 11 über die Auflagefläche 81 für den Schlauch 10 hinausragen. Das Schliessstück 31' ragt bezüglich der axialen Richtung um einen Betrag 12<11 nach unten über die Querstange heraus und ist in der dazu senkrechten Richtung so bemessen, dass es zwischen die beiden Vorsprünge 85 passt. Der minimal mögliche Abstand I1-I2 zwischen dem Schliessstück 31' und der Auflagefläche 81 des Gegenstücks 8 ergibt sich, wenn die Querstange 34 auf den beiden Vorsprüngen 85 aufliegt.

Um das Schliessorgan 3 aus der Offenstellung in die Schliessstellung oder aus der Schliessstellung in die Offenstellung zu bewegen, werden in analoger Weise wie vorne für das erste Ausführungsbeispiel beschrieben die Spulen 4 mit einem positiven oder einem negativen Strom beaufschlagt. In den beiden Gleichgewichtslagen (Offenstellung, Schliessstellung) wird das Schliessorgan 3 - ohne die Zufuhr von Strom - von den beiden permanentmagnetischen Haltern 2a,2b gegen die Federkraft der Federn 6' und der "Schlauchfeder" passiv magnetisch gehalten.

Fig. 7 zeigt in einer schematischen Darstellung ein Ausführungsbeispiel eines Fluidsystems für biologische Fluide, welches die Kombination einer Pumpvorrichtung 100 zum Fördern eines biologischen Fluids mit einer Klemmvorrichtung 1 gemäss der Erfindung umfasst. Das Fluidsystem ist beispielsweise eine Herz-Lungen-Maschine zum Aufrechterhalten des Blutkreislaufs im Körper eines Patienten. Die Pumpvorrichtung 100 ist eine Blutpumpe, deren Eingang mit dem Blutkreislauf des Patienten verbunden ist, sodass das Blut aus dem Körper zu der Blutpumpe 100 strömen kann, wie dies der Pfeil E andeutet. Die Blutpumpe 100 fördert das Blut in einem mit ihrem Ausgang verbundenen Schlauch 10. Ferner ist eine
erfindungsgemässe Klemmvorrichtung 1 vorgesehen, die gemäss dem ersten oder dem zweiten Ausführungsbeispiel ausgestaltet sein kann. Der Schlauch 10 wird durch die Klemmvorrichtung 1 hindurchgeführt und liegt auf der Auflagefläche 81 des Gegenstücks 8 auf. Stromabwärts der Klemmvorrichtung 1 ist der Schlauch 10 mit dem Blutkreislauf des Patienten verbunden, sodass das geförderte Blut dem Körperkreislauf zugeführt wird, wie dies der Pfeil K andeutet.

Stromabwärts des Ausgangs der Blutpumpe 100 und stromaufwärts der Klemmvorrichtung 1 ist ein Blasendetektor 101 vorgesehen, der überwacht, ob in dem Blut, welches durch den Schlauch 10 strömt, Luftblasen vorhanden sind. Der Blasendetektor ist über eine Signalleitung 102 mit der oder den Ansteuer- und Versorgungseinrichtung(en) 11a,11b der Klemmvorrichtung 1 verbunden. Die Ansteuer- und Versorgungseinrichtungen 11a,11b sind über Leitungen 103 mit den Spulen der Klemmvorrichtung 1 verbunden.

Während des normalen Betriebs befindet sich das Schliessorgan 3 der Klemmvorrichtung 1 in seiner Offenstellung, sodass das geförderte Blut in den Körper des Patienten strömen kann. Detektiert der Blasendetektor eine Luftblase in dem Schlauch 10, so übermittelt er ein Signal an die Ansteuerund Versorgungseinrichtungen 11 a, 11b, die dann einen entsprechenden Strom in die Spulen der Klemmvorrichtung 1 einspeisen, sodass das Schliessorgan 3 in die Schliessstellung bewegt wird und den Schlauch 10 zwischen dem Schliessstück 31,31' und der Auflagefläche 81 abklemmt. Dann ist die Strömungsverbindung unterbrochen und es kann kein Blut mehr durch die Klemmvorrichtung 1 hindurchströmen. Da mit der erfindungsgemässen Klemmvorrichtung 1 problemlos Schaltzeiten von weniger als 100 Millisekunden und insbesondere von höchsten 80 Millisekunden realisierbar sind, kann die Klemmvorrichtung 1 die Strömungsverbindung für das Blut schliessen, bevor die von dem Blasendetektor 101 detektierte Blase die Klemmvorrichtung 1 passiert hat. Somit wird sicher vermieden, dass die Luftblase in den Körper des Patienten gelangt. Nach Entfernen der Luftblase, beispielsweise durch Entlüften des Schlauchs 10, können die Ansteuer- und Versorgungvorrichtungen 11a, 11b durch entsprechende Strombeaufschlagung der Spulen der Klemmvorrichtung 1 das Schliessorgan wieder in die Offenstellung schalten.

Da die erfindungsgemässe Klemmvorrichtung 1 nur für das Umschalten zwischen der Offenstellung und der Schliessstellung Strom benötigt, nicht aber für das Halten des Schliessorgans 3 in den stabilen Gleichgewichtslagen (Offen- und Schliessstellung) hat die Klemmvorrichtung 1 einen sehr geringen Energie- bzw. Stromverbrauch und eignet sich somit insbesondere auch für portable Systeme.

Zudem kann die Klemmvorrichtung 1 sehr schnell und in einfacher Weise manuell betätigt werden. Durch Drücken auf den Griff 33;33' bzw. Ziehen an dem Griff 33;33' lässt sich das Schliessorgan 3 aus der Offen- in die Schliessstellung bzw. umgekehrt bewegen. Somit kann die Klemmvorrichtung 1 auch bei einem Ausfall der Stromversorgung oder bei einem Fehler in den Spulen sehr rasch und zuverlässig betätigt werden.

Es versteht sich, dass ausser dem Blasensetektor 101 auch andere Überwachungsmittel in dem Fluidsystem vorhanden sein können, die mittels eines Steuersignals ein Umschalten der Klemmvorrichtung 1 aus der Offenin die Schliessstellung und umgekehrt bewirken können

Natürlich eignet sich die erfindungsgemässe Klemmvorrichtung 1 auch für Fluidsysteme, in denen andere vorzugsweise biologische Flüssigkeiten oder Fluide transportiert werden als Blut, z. B. Infusionslösungen oder Nährlösungen in Bioreaktoren.

## Patentansprüche

1. Klemmvorrichtung zum Abklemmen eines Schlauchs in einem Fluidsystem für biologische Fluide, insbesondere Blut, mit einem bewegbar angeordneten Schliessorgan (3), welches ein Schliessstück (31,31') zum Abklemmen des Schlauches (10) umfasst, mit einer permanentmagnetischen Halteeinrichtung (2), welche derart angeordnet und ausgestaltet ist, dass sie das Schliessorgan (3) gegen eine Kraft in zwei verschiedenen stabilen Gleichgewichtslagen, nämlich einer Offenstellung und einer Schliessstellung, halten kann, ohne dass der permanentmagnetischen Halteeinrichtung (2) für das Halten in der jeweiligen Gleichgewichtslage Energie zugeführt werden muss, sowie mit Betätigungsmitteln (4;4a,4b;33;33'), um das Schliessorgan (3) aus der Offenstellung in die Schliessstellung zu bewegen.

2. Klemmvorrichtung nach Anspruch 1, bei welcher die Betätigungsmittel (4;4a,4b) mindestens eine Spule umfassen, welche so angeordnet ist, dass sie auf das Schliessorgan (3) eine in Richtung auf die Schliessstellung oder in Richtung auf die Offenstellung wirkende elektomagnetische Kraft ausüben kann.

3. Klemmvorrichtung nach einem der vorangehenden Ansprüche, bei welcher das Schliessorgan (3) zwei Schenkel (32a,32b) sowie eine Querstange (34) umfasst, wobei die Schenkel (32a,32b) beabstandet zueinander mit der Querstange (34) verbunden sind, bei welcher die permanentmagnetische Halteeinrichtung (2) zwei permanentmagnetische Halter (2a,2b) umfasst, von denen jeder jeweils einen Schenkel (32a,32b) umgibt, und bei welcher das Schliessstück (31') an der Querstange (34) zwischen den beiden Schenkeln (32a,32b) angeordnet ist.

4. Klemmvorrichtung nach Anspruch 3, bei welcher für jeden Schenkel (32a,32b) des Schliessorgans (3) mindestens eine den Schenkel (32a,32b) umgebende Spule (4) zum Ausüben einer elektromagnetischen Kraft auf den Schenkel (32a,32b) vorgesehen ist.

5. Klemmvorrichtung nach einem der vorangehenden Ansprüche, bei welchem für das Schliessorgan (3) oder für jeden Schenkel (32a,32b) des Schliessorgans (3) zwei separate Spulen (4a,4b) als Betätigungsmittel vorgesehen sind, wobei für jede der beiden separaten Spulen 4a,4b jeweils eine getrennte Ansteuer- und Versorgungsvorrichtung (11a,11b) vorgesehen ist.

6. Klemmvorrichtung nach einem der vorangehenden Ansprüche, bei welcher die Betätigungsmittel mechanische Betätigungsmittel (33;33') umfassen.

7. Klemmvorrichtung nach einem der vorangehenden Ansprüche, mit mindestens einem auf das Schliessorgan (3) einwirkenden Federelement (6;6'), welches so angeordnet ist, dass die Federkraft der magnetischen Kraft entgegenwirkt, welche die permanentmagnetische Haltevorrichtung (2) auf das Schliessorgan (3) ausübt.

8. Klemmvorrichtung nach einem der vorangehenden Ansprüche mit einem Gegenstück (8) zum Auflegen des Schlauchs (10), welches so angeordnet ist, dass der Schlauch (10) zwischen dem Schliessstück (31,31') des Schliessorgans (3) und dem Gegenstück (8) eingeklemmt werden kann.

9. Klemmvorrichtung nach Anspruch 8, bei welcher das Gegenstück (8) federnd und verstellbar gelagert ist.

10. Klemmvorrichtung nach Anspruch 8 oder 9, mit Sicherungsmitteln (85,312a,31'), welche so ausgestaltet sind, dass der Abstand zwischen dem Gegenstück (8) und dem mit ihm zusammenwirkenden Schliessstück (31,31') des Schliessorgans (3) stets grösser ist als ein Minimalwert (I1-I2).

11. Kombination einer Pumpvorrichtung (100) zum Fördern eines biologischen Fluids, insbesondere zum Fördern von Blut, mit einer Klemmvorrichtung (1) gemäss einem der vorangehenden Ansprüche.
